# EUROPEAN PATENT SPECIFICATION

(11) **EP 1 675 835 B1**
(45) Date of publication and mention of the grant of the patent: **21.09.2011**
(21) Application number: 03738352.8
(22) Date of filing: 07.07.2003
(51) Int. Cl.: C07D 241/18, C07D 403/06, A61K 31/496, A61P 35/00, C07K 5/12

(54) **METHODS OF PREPARATION OF DEHYDRO-DIKETOPIPERAZINES IN LIQUID PHASE OR ON SOLID SUPPORTED PHASE**
VERFAHREN ZUR HERSTELLUNG VON DEHYDRO-DIKETOPYPERAZINEN IN FLÜSSIGER PHASE ODER PER FESTPHASENSYNTHESE
PROCEDES POUR L'ELABORATION DE DESHYDRO-DICETOPIPERAZINES EN PHASE LIQUIDE OU SOLIDE

(30) Priority: 03.07.2003 GR 20030100287
(43) Date of publication of application: 05.07.2006
(73) Proprietor: pro-ACTINA S.A., 19400 Koropi Attikis (GR)
(72) Inventor: COULADOUROS, Elias, 154 51 New Psychico, Athens (GR); MAGOS, Alexandros, 175 62 Palaio Faliro, Athens (GR); STRONGILOS, Alexandros, Athens (GR)
(74) Representative: Becker, Eberhard
(86) International application number: PCT/GR2003/000028
(87) International publication number: WO 2005/003102

(56) References cited:
- EP-A- 1 264 831
- US-B1- 6 358 957
- DEL FRESNO M ET AL: "Solid-Phase Synthesis of Diketopiperazines, Useful Scaffolds for Combinatorial Chemistry" TETRAHEDRON LETTERS, ELSEVIER SCIENCE PUBLISHERS, AMSTERDAM, NL, vol. 39, no. 17, 23 April 1998 (1998-04-23), pages 2639-2642, XP004112118 ISSN: 0040-4039
- JAMES I W: "Linkers for Solid Phase Organic Synthesis" TETRAHEDRON, ELSEVIER SCIENCE PUBLISHERS, AMSTERDAM, NL, vol. 55, no. 16, 16 April 1999 (1999-04-16), pages 4855-4946, XP004161079 ISSN: 0040-4020
- JOHNSON C R ET AL: "Solid Phase Synthesis of Alkenes Using The Horner-Wadsworth-Emmons Reaction and Monitoring by Gel Phase P NMR" TETRAHEDRON LETTERS, ELSEVIER SCIENCE PUBLISHERS, AMSTERDAM, NL, vol. 36, no. 51, 18 December 1995 (1995-12-18), pages 9253-9256, XP004026728 ISSN: 0040-4039

## Description

### FIELD OF THE INVENTION

The present invention relates to the fields of synthetic organic chemistry, combinatorial chemistry and medicinal chemistry. It relates to the design and preparation of compounds potentially useful for the treatment of cancer.

### BACKGROUND OF THE INVENTION

Diketopiperazines of natural or unnatural amino-acids constitute an important chemical class including some biologically active members. Recently, it has been reported that tryprostatins A and B (which are diketopiperazines consisting of proline and isoprenylated tryptophan residues), and five other structurally-related diketopiperazines, inhibited cell cycle progression in the M phase [Cui, C. et al., J. Antibiotics (1996), 49, 527-533; Cui, C. et al. J. Antibiotics (1996), 49, 534-540] and that these compounds also affect the microtubule assembly, [Usui, T. et al. Biochem J. (1998), 333, 543-548; Kondon, M. et al. J. Antibiotics (1998), 51, 801-804]. Furthermore, naturally occurring dehydro-diketopiperazines, such as phenylahistin, aurantiamine, roquefortine, isoroquefortine, viridamine etc, have been found to exhibit anticancer and neurotoxic activities [Aninat C., Hayashi Y., Andre F., Delaforge M. Chem. Res. Toxicol. (2001), 14, 1259-1265; Kanoh K., Kohno S., Katada J., Takahashi J., Uno I., Hayashi Y. Bioorg. Med Chem. (1999), 7, 1451-1457; Kanoh K., Kohno S., Asari T., Harada T., Katada J., Muramatsu M., Kawashima H., Sekiya H., Uno I. Bioorg. Med. Chem. Lett. (1997), 7, 22, 2847-2852; Kanoh K., Kohno S., Katada J., Takahashi J., Uno I. J. Antibiotics (1999), 52 (2), 134-141]. A few patents covering the isolation, biological activities and derivatization of this class of compounds, especially for phenylahistine, appear in the literature [Fukumoto K., Kohno S., Kanoh K., Asari T., Kawashima H., Sekiya H., Ohmizo K., Harada T., Knobbe Martens Olson & Bear LLP, (San Diego, CA) US 6,358,957 March 19, 2002; Fukumoto K., Kohno S., Kanoh K., Asari T., Kawashima H., Sekiya H., Ohmim K., Harada T., Knobbe Martens Olson & Bear LLP, (San Diego, CA) US 2002143021 October 3, 2002].
EP-B-1 264 831 discloses a cell division inhibitor and a method of producing them using enzymes, wherein as cell division inhibitors, dehydro-diketopiperazines are used.
All the reported in the literature preparation methods of dehydro-diketopiperazines so far, consist of a direct coupling between the diketopiperazine core and a formyl-derivative. These methods have very low chemical yields resulting also in almost racemic mixtures. However, a general convergent synthetic method is not documented. The advantage of a convergent strategy for the preparation of the main molecular framework of a pharmacophoric structure, such as dehydro-diketopiperazines, is that it can be used for the preparation of libraries of potentially bioactive derivatives.

The main novelty of the presented methodology pertains the strategic disconnection of the target molecules into three components: an amino-acid, a phosphinylglycine derivative and an aldehyde. This strategy provides a highly convergent and short procedure of high yielding reactions, utilizing mild coupling conditions of the component moieties, which prevent any racemization of the final products.

### BRIEF DESCRIPTION OF THE DRAWINGS

The accompanying drawings, which are incorporated in and form part of the specification, merely illustrate embodiments of the present invention. Together with the remainder of the specification, they are meant to serve to explain preferred modes of making certain compounds of the invention to those of skilled in the art. In the drawings:
FIG. 1. Illustrates the liquid chemical route for the synthesis of dehydro-diketopiperazines.
FIG. 2. Illustrates the general chemical route for the preparation of solid supported phosphonates.
FIG. 3. Illustrates the solid phase synthetic route for the preparation of dehydro-diketopiperazines.
FIG. 4, 5, 6, 7. Illustrates examples of derivatives of dehydro-diketopiperazines, the preparation of which is described herein.

### DETAILED DESCRIPTION

This invention provides a general method for the synthesis of dehydro-diketopiperazines represented by the following general disconnection scheme:

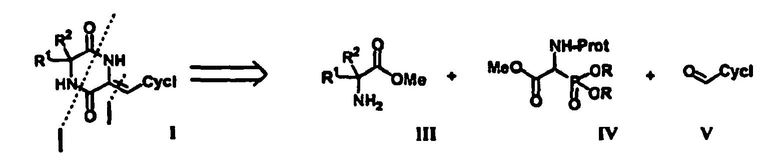

The main novelty of the presented methodology pertains the strategic disconnection of the target molecules into three components: an aminoacid, a phosphinylglycine derivative and an aldehyde. This strategy provides a highly convergent and short procedure of high yielding reactions and on the other hand, the mild coupling conditions of the component moieties prevent any racemization of the final products.

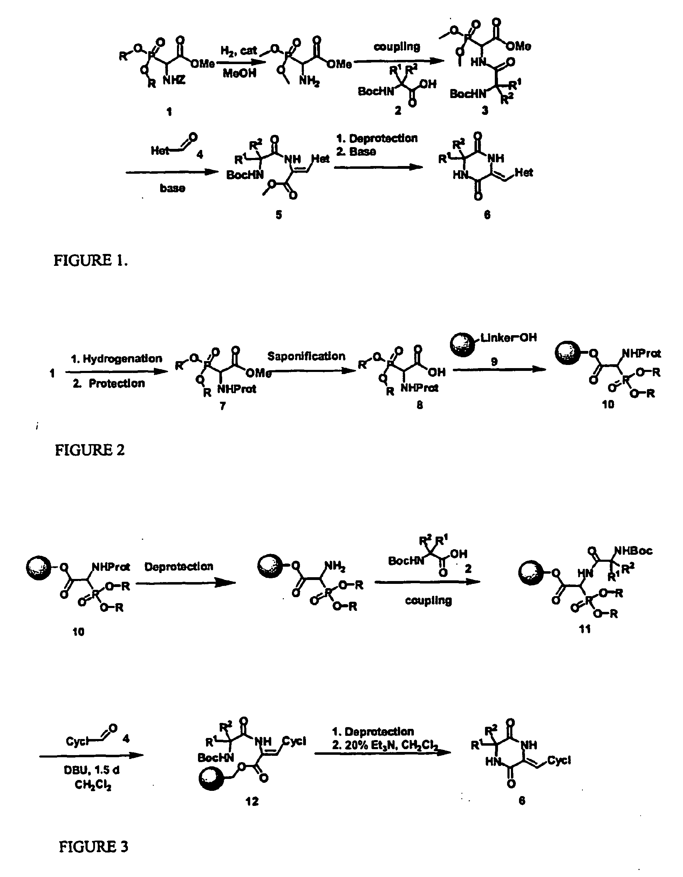

As described in Fig. 1, phosphinylglycinate **1** [prepared according to Schmidt U., Lieberknecht A., Wild J. Synthesis (1984), 53-60], after hydrogenation, is coupled under standard conditions (EDC.HCl/HOBt, DCC/HOBt, Pybop etc.) with *N*-protected aminoacid bearing the R¹ and R² substitutents, **2.** The derived phosphinyl derivative **3,** is subsequently coupled with the formyl derivative of *Cycl*-substitutent **4,** utilizing mild alkaline conditions (e.g. Et₃N, DBU, Masamune protocol, Cs₂CO₃, LDA, etc) affording protected *seco*-acid **5.** The protected amine of the latter compound is released and the pH of the reaction mixture is subsequently maintained to be mildly basic (e.g. using Et₃N) favoring an intramolecular attack to the carboxylate carbon. Thus, the target molecule **6** is formed within **4** steps in high yield and less than 5% racemization or *Z,E*-variation at the double bond. Examples 1, 2 and 3 refer to this Figure.

Loading of a phosphinylglycine derivative on a resin is described on Fig. 2. Accordingly, compound **1** is hydrogenated, the amino group is subsequently protected (e.g. as Boc, Fmoc, Teoc, Bpoc, etc), and the ester functionality is saponified to yield acid **8.** The latter is loaded on a -OH terminated resin using conventional coupling conditions (e.g. dehydration, Mitsunobu protocol, acid activation, etc) to provide the target supported key-intermediate **10.** The chemistry described on Figure 1 may then be applied on **10** to afford all compounds described in this patent.

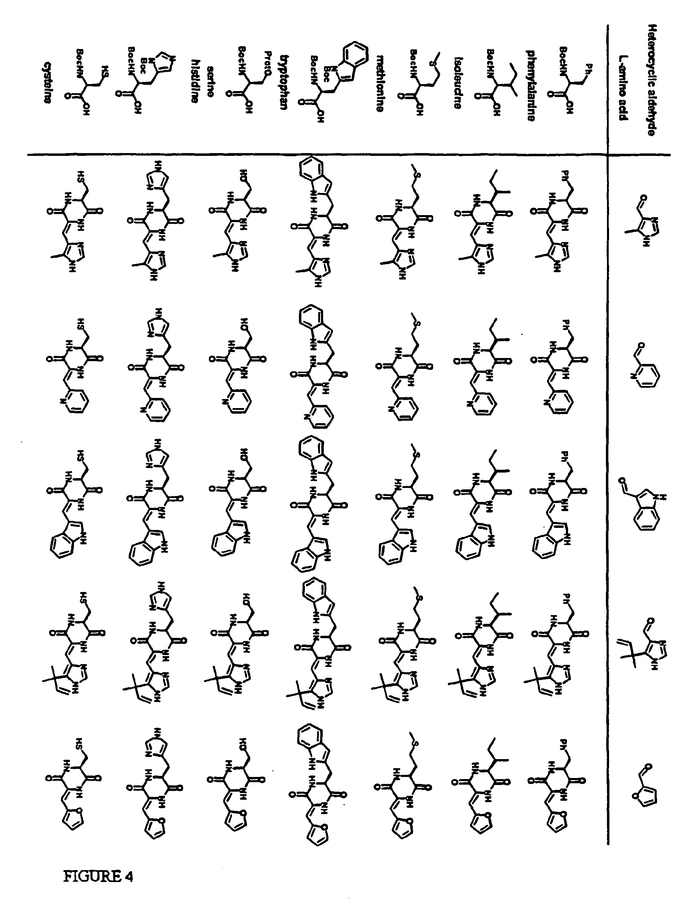

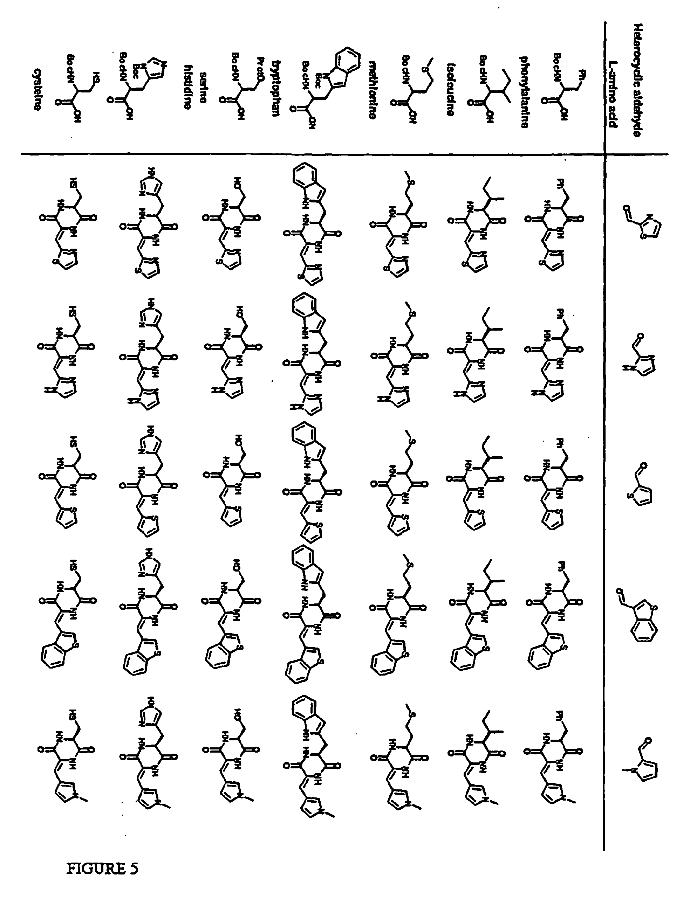

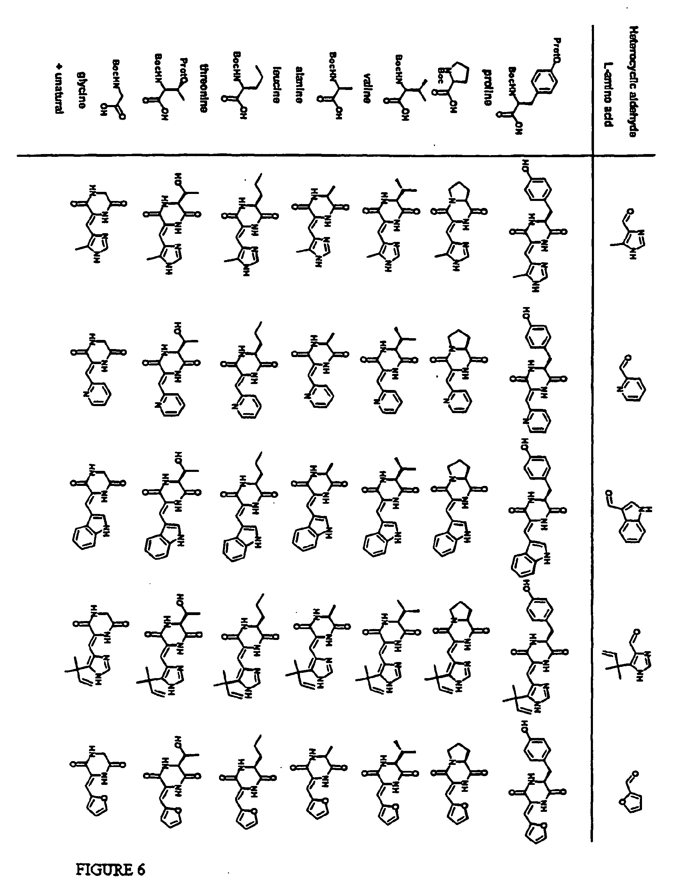

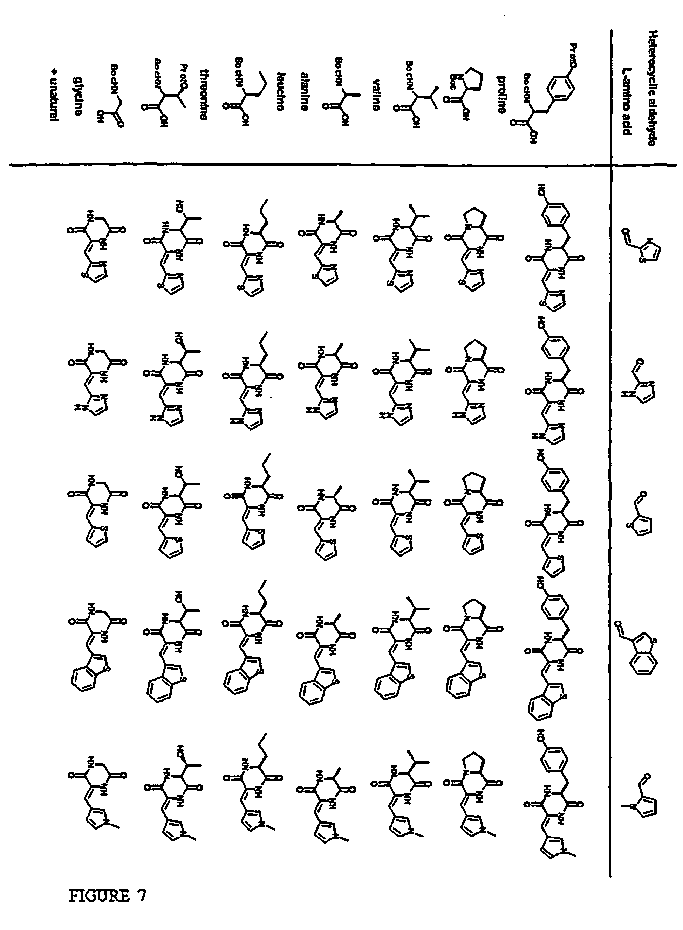

As it is shown on Fig. 3, deprotection of the amine moiety of **10** and conventional coupling with the free acid of amino acid **2,** provides the supported intermediate **11,** which is subsequently coupled with formyl derivative **4** using the same conditions described on Fig. 1, to furnish loaded intermediate **12.** The latter, after deprotection of the amine group and intramolecular amidation under mild alkaline conditions is transformed to the target dehydro-diketopiperazine with concomitant cleavage from the resin. A few examples of possible R', R² and *Cycl-* variations are given on Fig. 4, 5, 6, and 7. The procedure of loading the phosphinylglycinate and the formation of the library, are given on Examples 4-9. Among the final compounds several are already known natural products (e.g. phenylahistin, aurantiamine, viridamine). However, for none of them is anywhere documented to be synthesized in a similar manner. Related patents for the isolation, biological evaluation and derivatization of relevant known natural products, the synthesis of which has been described in this patent, were given previously within this section.

### Examples

### EXAMPLE 1

To a solution of methyl-2-(benzyloxycarbonylamino)-2-(dimethoxyphosphinyl)-acetate, **1,** (1 g, 3.02 mmoles) in methanol (55 ml) was added a catalytic amount of 10% of palladium activated on carbon (100 mg) and the mixture was stirred for 4h under hydrogen atmosphere. The reaction mixture was filtered through a celite pad and the solvent was removed under reduced pressure. The crude mixture of methyl-2-amino-2-(dimethoxyphospinyl)-acetate was subsequently dissolved in CH₂Cl₂ (40 ml) under argon and *N*-Boc protected *L*-phenylalanine (1.04 g, 3.92 mmoles) was added. The mixture was cooled to 0°C, HOBT (530.3 mg 3.92 mmoles) and EDC.HCl (752.5 mg, 3.92mmoles) were added successively. The reaction mixture was stirred for 3h at room temperature, then the solvent was removed under reduced pressure, the residue was dissolved in ethyl acetate (40 ml) and washed with saturated sodium hydrogen carbonate (30 ml), H₂O (30 ml), brine (30 ml), dried over anhydrous Na₂SO₄ and concentrated in vacuum. The crude mixture was subjected to flash chromatography to afford the amide **3** as light yellow foam (1.073 g, 80%).

[a]²⁵D = -11.02 (c=2.9 CHCl₃);
¹H NMR (250 MHz) 25°C, CDCl₃: δ 7.38-7.15 (m, 5H), 5.26-5.10 (dd, 1H, *J*= 21.9 Hz, 8.93 Hz), 5.04-4.86 (m, 2H), 4.54-4.36 (m, 2H), 3.87-3.68 (m. 6H), 3.79 (s, 3H), 3.23-2.94 (m, 2H), 1.39 (s, 9H);
¹³C NMR (62.9 MHz) 25°C, CDCl₃: δ 171.6, 171.5, 166.8, 166.7, 166.6, 155.3, 136.5, 132.1, 131.9, 129.3, 128.5, 128.3, 126.8, 79.9, 64.2, 55.4, 54.2 (d, *J_{COP}* = 7.7 Hz), 54.01 (d, *J_{COP}* = 6.8 Hz), 53.2, 50.1 (d, *J_{CP}* = 150.89 Hz), 38.2, 28.2;
FTIR: (KBr) v: 3420, 1750, 1720 cm⁻¹

### EXAMPLE 2

To a mixture of *N*-*p*-toluenesulfonylo-5-(1,1-dimethyl-2-propenyl)-imidazole-4-carboxaldehyde, 4 (46 mg, 0.145 mmoles) and phosphonate derivative **3** (96.3 mg, 0.217 mmoles) in dry CH₂Cl₂ (0.5 ml) under argon, at 0°C, was added DBU (43.2 µl, 0.289 mmoles). After being stirred at room temperature for 16 hours, the reaction mixture was quenched with saturated ammonium chloride (5 ml) and extracted with ethyl acetate (2 x 10 ml). The organic layer was washed with brine (10 ml), dried over anhydrous Na₂SO₄ and concentrated in vacuum. The crude mixture was subjected to flash chromatography to afford the alkene **5** as a yellow oil (36.2 mg, 51.7%).

[a]²⁵D = +13.42 (c=2.23 CHCl₃);
¹H NMR (250 MHz) 25°C, CDCl₃: δ 11.10 (br s, 1H), 9.21 (br s, 1H), 8.0 (br s, 1H), 7.42-7.19 (m, 5H), 6.78 (s, 1H), 6.28-5.93 (m, 1H), 5.30-5.05 (m, 2H), 4.49 (br s, 1H), 4.45-4.10 (m, 1H), 3.42-2.93 (m, 2H), 1.48 (s, 6H), 1.41 (s, 9H);
¹³C NMR (62.9 MHz) 25°C, CDCl₃: δ 170.5, 165.7, 155.9, 145.7, 136.2, 130.9, 129.4, 128.8, 128.6, 127.6, 126.9, 112.2, 80.6;
FTIR: (KBr) v: 3420, 1750, 1600 cm⁻¹

### EXAMPLE 3

To a solution of **5** (28.6 mg, 0.051 mmoles) in dry CH₂Cl₂ (3.4 ml) under argon at 0°C, was added trifluoroacetic acid (0.85 ml). The ice-bath was removed and the reaction was stirred for 2 hours at room temperature. The solvent is removed under reduced pressure and the crude amine was used in the next step without further purification.

¹H NMR (250 MHz) 25°C, MeOD: δ 8.74 (s, 1H), 7.52 (s, 1H), 7.50-7.38 (m, 5H), 6.21 (dd, 1H, *J*= 16.9, 10.6 Hz), 5.35 (d, 1H, *J*= 10.1 Hz), 5.32 (d, 1H, *J*= 17.4 Hz), 4.25-4.10 (m, 1H), 3.54 (dd, 1H, *J*= 14.7,5.0 Hz), 3.11 (dd, 1H, *J*= 14.2, 9.2 Hz), 1.64 (s, 3H), 1.63 (s, 3H).

This amine was dissolved in dry CH₂Cl₂ (4.1 ml) under argon and triethylamine (20% in CH₂Cl₂) was added at room temperature. After the completion of the reaction (2h) an aqueous saturated ammonium chloride solution (10 ml) wass added and the mixture and the mixture was extracted with chloroform (2 x 10 ml). The combined organic layers were washed with brine (10 ml), dried over anhydrous Na₂SO₄ and concentrated in vacuum. The mixture was subjected to flash chromatography to afford the natural product (-)- phenylahistin **6**, as a white solid (16.1 mg, 90% 2 steps).
mp: 227-229°C; [a]²⁵_{D} = -185 (c=0.1 MeOH);
¹H NMR (250 MHz) 25°C, CDCl₃: δ 12.00 (br s, 1H), 9.14 (br s, 1H), 7.55 (s, 1H), 7.42-7.19 (m, 5H), 6.88 (s, 1H), 6.02 (dd, 1H, *J*= 17.5, 10.8 Hz), 5.76 (br s, 1H), 5.20 (d, 1H, *J*= 10.8 Hz), 5.15 (d, 1H, *J*= 17.5 Hz), 4.34 (ddd, 1H, *J*= 10.1, 3.4, 2.3 Hz), 3.50 (dd, 1H, *J=* 13.8, 3.4 Hz), 2.95 (dd, 1H, *J*= 13.8, 10.1 Hz), 1.50 (s, 6H);
¹³C NMR (62.9 MHz) 25°C, CDCl₃: δ 164.7, 159.9, 144.6, 136.6, 135.5, 132.4, 132.2, 129.5, 129.1, 127.5, 123.8, 113.5, 105.4, 57.2, 41.3, 37.6, 27.9;
FTIR: (KBr) v: 3400, 1760, 1600 cm⁻¹

### EXAMPLE 4

To a solution of methyl-2-(benzyloxycarbonylamino)-2-(dimethoxyphosphinyl)-acetate, 1, (916 mg, 2.77 mmoles) in methanol (40 ml) was added a catalytic amount of 10% of palladium activated on carbon (91.6 mg) and the mixture was stirred for 4h under hydrogen atmosphere. The reaction mixture was filtered through a celite pad and the solvent was removed under reduced pressure. The crude mixture of methyl-2-amino-2-(dimethoxyphospinyl)-acetate was subsequently dissolved in CH₂Cl₂ (4.7 ml) under argon. To this solution was added di-t-butyl dicarbonate (784.6 mg, 3.6 mmole) and was stirred for 24h at room temperature. The mixture was then washed with 1N NaHSO₄ (50 ml), with saturated NaHCO₃ (50 ml), brine (50 ml), dried over anhydrous Na₂SO₄ and concentrated in vacuum. The mixture was subjected to flash chromatography to afford methyl-2-(*t*-butoxycarbonylamino)-2-(dimethoxyphosphinyl)-acetate, 7 as a white solid (675.8 g, 82.2%).
mp: 47-48 °C;
¹H NMR (250 MHz) 25°C, CDCl₃: δ 5.35 (br d, 1H, *J*= 8.9 Hz), 4.87 (br dd, 1H, *J*= 23.1, 8.9 Hz), 3.84 (d, 3H, *J*= 2.6 Hz), 3.82 (s, 3H), 3.79 (d, 3H, *J*= 2.3 Hz), 1.44 (s, 9H);
¹³C NMR (62.9 MHz) 25°C, CDCl₃: δ 167.2, 154.8, 80.0, 53.6, 53.5, 52.6, 50.3, 27.8; FTIR: (KBr) v: 3350,1745 cm⁻¹

### EXAMPLE 5

To a stirred solution of methyl-2- (*t*-butoxycarbonylamino)-2-(dimethoxyphosphinyl)-acetate **7**, (680 mg, 2.29 mmoles) in MeOH (10 ml) at 0°C, was added dropwise an aqueous solution of KOH (1N, 3.4 ml). The ice-bath was removed and the reaction was stirred for 2.5 hours at room temperature. The most of the organic solvent was removed under reduced pressure and the mixture is dissolved in H₂O (15 ml). The aqueous layer was washed once with ethyl acetate (15 ml), acidified with concentrated HCl to pH=2-3 and the product was extracted exhaustively with ethyl acetate (5 x 40 ml). The organic solvent is dried over anhydrous Na₂SO₄ and concentrated in vacuum to afford the desired product t-butoxycarbonylamino-2-dimethoxyphosphinyl-acetic acid, **8,** as a white solid (551 mg, 85% yield).
mp: 155-156 °C;
¹H NMR (250 MHz) 25°C, CDCl₃: δ 10.93 (br s, 1H), 5.71 (br d, 1H, *J*= 8.9 Hz), 4.90 (dd, 1H, *J*= 22.1, 8.9 Hz), 3.79 (t, 6H, *J*= 10.1 Hz), 1.39 (s, 9H);
¹³C NMR (62.9 MHz) 25°C, CDCl₃: δ 167.9, 155.1, 80.7, 54.6 (d, *J_{COP}* = 6.8 Hz), 54.4 (d, *J_{COP}* = 6.8 Hz), 51.5 (d, *J_{CP}* = 150.9Hz), 28.1

### EXAMPLE 6

Dried resin **9** (334 mg, 0.642mmol/gr) was suspended in a 4:1 mixture of dry CH₂Cl₂ / DMF (9ml) under argon and shaked for 20 minutes at room temperature. *t-*Butoxycarbonyl-amino-2-dimethoxyphosphinyl-acetic acid, **8**, (334 mg, 5 eq) was added and the solution was cooled at 0°C. Dicyclohexylcarboxycarbodiimide (265 mg, 6eq) and a catalytic amount of (4)-dimethylaminopyridine (13.1 mg, 0.5 eq) were added successively to the suspension . The mixture was shaked at 25°C for 2 days, then filtered and washed sequentially with CH₂Cl₂ (2 x 20 ml), MeOH (2 x 20 ml), H₂O (2 x 20 ml), CH₂Cl₂ (2 x 20 ml), MeOH (2 x 20 ml), CH₂Cl₂ (2 x 20 ml), MeOH (2 x 20 ml). The resin **10** was dried under vacuum for 24 h.
FTIR: (KBr) v: 3653.4, 3446.1, 3069.1, 3031.5, 2927.8, 2847.7, 1947.7, 1872.3, 1811.1, 1754.5, 1721.5, 1608, 1500, 1457, 1372.9, 1311.6, 1269.2, 1156.1, 1028.9, 845.1, 816.9, 750.9, 699.1 cm⁻¹

### EXAMPLE 7

The phosphinyl resin **10** (301 mg, 0.548 mmol/gr) was suspended in anhydrous CH₂Cl₂ (5 ml) and an excess of (+)-10-camphorsulfonic acid (12eq) was added. The suspension was shaked at 25°C for 2 days under argon atmosphere. The resin was filtered and washed sequentially with CH₂Cl₂ (2 x 20 ml), MeOH (20 ml), CH₂Cl₂ (2 x 20 ml).

Then the resin was suspended in anhydrous CH₂Cl₂ (7 ml), under argon atmosphere and *N*-Boc protected *L*-phenylalanine (200 mg, 4.5 eq) was added. An excess HOBT (170 mg, 6.5 eq) and EDC.HCl (240 mg, 6.5 eq) were added. Finally Et₃N (34 µl, 1.3e q) was added and the suspension was shaked for 36 hours at room temperature. The resin 11 is filtered, washed sequentially with CH₂Cl₂ (2 x 20 ml), MeOH (2 x 20 ml), CH₂Cl₂ (2 x 20 ml), MeOH (2 x 20 ml) CH₂Cl₂ (2 x 20 ml), MeOH (2 x 20 ml) and dried under vacuum for 24 h.

FTIR: (KBr) v: 3656, 3436, 3298, 3064, 3028, 2918, 2849, 1949, 1876, 1812, 1753, 1721, 1689, 1606, 1501, 1451, 1373, 1256, 1162, 1023, 845, 822, 745, 708 cm⁻¹

### EXAMPLE 8

The phoshinyl resin **11** (113.0 mg, 0.507 mmoles/gr) was suspended in anhydrous CH₂Cl₂ (2.5 ml) under argon atmosphere. *N*-*p*-toluenesulfonylo-5-(1,1-dimethyl-2-propenyl)-imidazole-4-carboxaldehyde, **4,** (36,4 mg, 2 eq) was added and the suspension was cooled at 0 °C. An excess of DBU (26.0 µl, 3 eq) was added and the suspension was allowed to shake at 25°C for 36 hours. The resin, **12,** was filtered, washed sequentially with CH₂Cl₂ (2 x 20 ml), MeOH (2 x 20 ml), CH₂Cl₂ (2 x 20 ml), MeOH (2 x 20 ml) and dried under vacuum for 24 h.

FTIR: (KBr) v: 3452, 3064, 3029, 2926, 2864, 1948, 1876, 1810, 1729, 1641, 1605, 1494, 1458, 1374, 1245, 1169, 1031, 911, 844, 818, 755, 697 cm⁻¹

### EXAMPLE 9

Resin **12,** (102 mg, 0.498 mmol/gr) was suspended in anhydrous CH₂Cl₂ (1.7 ml) under argon atmosphere. (+)-10-camphorsulfonic acid (10 eq) was added at 25°C and the mixture was shaked for 2 days. The deprotected resin is filtered and washed sequentially with CH₂Cl₂ (2 x 15 ml), MeOH (15 ml), Et₃N (5% in CH₂Cl₂, 3 x 15 ml), CH₂Cl₂ (2 x 15 ml), MeOH (2 x 15 ml). Then resin was suspended in anhydrous CH₂Cl₂ (1.6 ml), and an excess of triethylamine (0.4 ml, 20% in CH₂Cl₂) was added. The suspension after vigorous stirring for 36 hours was filtered and washed with CH₂Cl₂ (2 x 15 ml) and MeOH (2 x 15 ml). The solvent was removed under reduced pressure and the residue was dissolved in CHCl₃ (15 ml). The crude mixture was extracted with NH₄Cl (15 ml), brine (15 ml), dried over anhydrous Na₂SO₄ and concentrated under vacuum. The mixture was then filtered through silica gel to afford the natural product (-)-phenylahistin, **6**, as a white solid (6 mg, 34% 6 steps).

mp: 227-229 °C; [a]²⁵_{D} = -185 (c=0.1 MeOH);
¹H NMR (250 MHz) 25°C, CDCl₃: δ 12.00 (br s, 1H), 9.14 (br s, 1H), 7.55 (s, 1H), 7.42-7.19 (m, 5H), 6.88 (s, 1H), 6.02 (dd, 1H, *J*= 17.5, 10.8 Hz), 5.76 (br s, 1H), 5.20 (d, 1H, *J*= 10.8 Hz), 5.15 (d, 1H, *J*= 17.5 Hz), 4.34 (ddd, 1H, *J*= 10.0, 3.4, 2.3 Hz), 3.50 (dd, 1H, *J*= 13.8, 3.4 Hz), 2.95 (dd, 1H, *J*= 13.8, 10.1 Hz), 1.50 (s, 6H);
¹³C NMR (62.9 MHz) 25°C, CDCl₃: δ 164.7, 159.9, 144.6, 136.6, 135.5, 132.4, 132.2, 129.5, 129.1, 127.5, 123.8, 113.5, 105.4, 57.2, 41.3, 37.6, 27.9

## Claims

1. A process for the preparation of dehydro-diketopiperazines in solution using the following sequence: coupling of the amine group of methyl ester of 2-dimethoxyphosphinyl-glycinate with the carboxylic group of an *N*-protected aminoacid with α,α'-substitutents R¹ and R² and successively coupling of the above formed compound with the formyl-*Cycl* derivative employing mild alkaline conditions and finally, deprotection of the *N*-protective group and use of mild alkaline conditions to yield the dehydro-diketopiperazine with the formation of an intramolecular amide bond said dehydro-diketopiperazines being represented by the following structure: 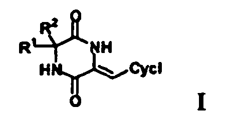 wherein:
R¹ and R² may be the same or different and each represents an optionally substituted alkyl, cycloalkyl, alkenyl, cycloalkenyl, alkoxy, alkyloxy, alkylthio, cycloalkoxy, acyloxy, aryl, substituted aryl, heteroaryl, substituted heteroaryl, amino, substituted amino, phenyl, and substituted phenyl groups, a hydrogen atom, a halogen atom, a cyano group, a nitro group, a hydroxyl group, a thiol group or a group representing the α,α'-substitutents of naturally occurring aminoacids, the configuration of the carbon atom bearing substitutents R¹ and R² may be *R*-, *S-* or racemic mixture;
*Cycl-* is a group represented by either of the following formulae: 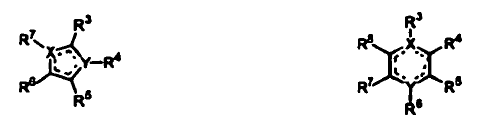
wherein:
R³ to R⁸ may be the same or different and each represents an optionally substituted alkyl, cycloalkyl, alkenyl, cycloalkenyl, alkoxy, alkyloxy, alkylthio, cycloalkoxy, acyloxy, aryl, substituted aryl, heteroaryl, substituted heteroaryl, amino, substituted amino, phenyl, and substituted phenyl groups, a hydrogen atom, a halogen atom, a cyano group, a nitro group, a thiol group or a hydroxyl group, one out of R³ to R⁸ represents the linking bond with the double bond of the diketopiperazine core described above, the dashed bond represents a bond selected from the group consisting of a single bond or a double bond and
X and Y may be the same or different and each represents a carbon atom or a nitrogen atom or an oxygen atom or a sulphur atom, the carbon, nitrogen or sulphur atoms may be optionally oxidized or substituted.

2. The process according to Claim 1 on a solid supported phosphinylglycinate for the preparation of single members or groups of dehydro-diketopiperazines in solid phase, wherein the solid supported phosphinylglycinate is represented by the general formula: 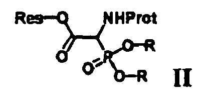 wherein:
Res represents any polymer support (resin),
Prot represents any NH-protective group
R represents alkyl or alkyloxyalkyl groups.

3. The preparation of a solid supported phosphinylglycinate of formula (II) as described in Claim 2, by the coupling of the carboxylate group of an *N*-protected phosphinylglycinate and an -OH terminated resin using conventional coupling conditions.

4. The use of the solid supported phosphinylglycinate of formula (II) described in Claims 2 and 3, for the preparation of dehydro-diketopiperazines described in Claim 1.

## Patentansprüche

1. Verfahren zur Herstellung von Dehydrodiketopiperazinen in Lösung unter Verwendung der folgenden Reihenfolge: Kopplung der Amingruppe des Methylesters von 2-Dimethoxyphosphinylglycinat mit der Carboxylgruppe einer *N*-geschützten Aminosäure mit α,α'-Substituenten R¹ und R² und der anschließenden Kopplung der vorangehend gebildeten Verbindung mit dem Formyl-*Cycl*-Derivat unter Einsatz von milden alkalischen Bedingungen und zum Schluss Entschützen der N-Schutzgruppe und Verwendung von milden alkalischen Bedingungen, um das Dehydrodiketopiperazin durch die Bildung einer intramolekularen Amidbindung zu ergeben, wobei die Dehydro-diketopiperazine durch die nachfolgende Struktur wiedergegeben werden: 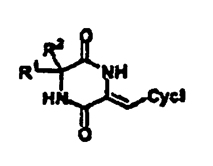 wobei:
R¹ und R² gleich oder unterschiedlich sein können und jeweils unabhängig voneinander für eine gegebenenfalls substituierte Alkyl-, Cycloalkyl-, Alkenyl-, Cycloalkenyl-, Alkoxy-, Alkyloxy-, Alkylthio-, Cycloalkoxy-, Acyloxy-, Aryl-, substituierte Aryl-, Heteroaryl-, substituierte Heteroaryl-, Amino-, substituierte Amino-, Phenyl- und eine substituierte Phenylgruppe, ein Wasserstoffatom, ein Halogenatom, eine Cyanogruppe, eine Nitrogruppe, eine Hydroxygruppe, eine Thiolgruppe oder eine Gruppe stehen, welche für die Substituenten der natürlich vorkommenden Aminosäuren steht, wobei die Konfiguration des Kohlenstoffatoms, das die Substituenten R¹ und R² trägt, in der *R*- oder *S*-Form oder als ein racemisches Gemisch vorliegen kann;
*Cycl*- eine Gruppe ist, die für eine der nachfolgenden Formeln dargestellt wird: 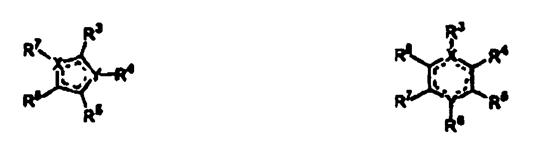
wobei:
R³ bis R⁸ gleich oder unterschiedlich sein können und jeweils unabhängig voneinander für eine gegebenenfalls substituierte Alkyl-, Cycloalkyl-, Alkenyl-, Cycloalkenyl-, Alkoxy-, Alkyloxy-, Alkylthio-, Cycloalkoxy-, Acyloxy-, Aryl-, substituierte Aryl-, Heteroaryl-, substituierte Heteroaryl-, Amino-, substituierte Amino-, Phenyl- und eine substituierte Phenylgruppe, ein Wasserstoffatom, ein Halogenatom, eine Cyanogruppe, eine Nitrogruppe, eine Thiolgruppe oder eine Hydroxygruppe stehen, wobei eines von R³ bis R⁸ für die verknüpfende Bindung mit der Doppelbindung von dem vorangehend beschriebenen Diketopiperazin-Grundgerüst steht, die gestrichelte Bindung für eine Bindung steht, die ausgewählt ist aus der Gruppe, die aus einer Einfachbindung oder einer Doppelbindung besteht, und
X und Y gleich oder unterschiedlich sein können und jeweils unabhängig voneinander für ein Kohlenstoffatom oder ein Stickstoffatom oder ein Sauerstoffatom oder ein Schwefelatom stehen, wobei die Kohlenstoff-, Stickstoff- oder Schwefelatome gegebenenfalls oxidiert oder substituiert sein können.

2. Verfahren nach Anspruch 1 an einem festphasengebundenen Phosphinglycinat zur Herstellung von einzelnen Mitgliedern oder Gruppen von Dehydrodiketopiperazinen in der Festphase, wobei das festphasengebundene Phosphinglycinat dargestellt ist durch die allgemeine Formel: 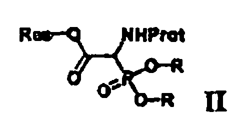 wobei:
Res irgendein polymeres Trägermaterial (Harz) darstellt,
Prot irgendeine NH-Schutzgruppe darstellt,
R Alkyl- oder Alkyloxyalkyl-Gruppen darstellt.

3. Herstellung eines festphasengebundenen Phosphinglycinats der Formel (II) nach Anspruch 2 durch die Kopplung der Carboxylat-Gruppe eines N-geschützten Phosphinglycinats und einer endständigen OH-Gruppe des Harzes unter Verwendung herkömmlicher Kopplungsbedingungen.

4. Verwendung des festphasengebundenen Phosphinglycinats der Formel (II) nach Anspruch 2 und 3 zur Herstellung von Dehydrodiketopiperazinen nach Anspruch 1.

## Revendications

1. Procédé de préparation de déhydro-dicétopipérazines en solution en utilisant la séquence suivante : couplage du groupe amine du méthyl ester de 2-diméthoxyphosphinyl-glycinate avec le groupe acide carboxylique d'un acide aminé N-protégé avec les α,α'-substituants R¹ et R² et couplage successif du composé formé ci-dessus avec le dérivé formyl-*Cycl* en utilisant des conditions alcalines modérées et finalement, déprotection du groupe *N*-protecteur et utilisation de conditions alcalines modérées pour donner la déhydro-dicétopipérazine avec la formation d'une liaison amide intramoléculaire,
lesdites déhydro-dicétopipérazines étant représentées par la structure suivante : 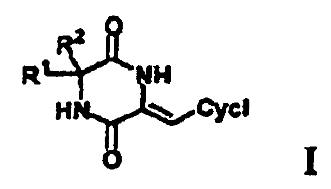 dans laquelle :
R¹ et R² peuvent être identiques ou différents et représentent chacun un groupe alkyle facultativement substitué, cycloalkyle, alcényle, cycloalcényle, alcoxy, alkyloxy, alkylthio, cycloalcoxy, acyloxy, aryle, aryle substitué, hétéroaryle, hétéroaryle substitué, amino, amino substitué, phényle, et phényle substitué, un atome d'hydrogène, un atome d'halogène, un groupe cyano, un groupe nitro, un groupe hydroxyle, un groupe thiol ou un groupe représentant les α,α'-substituants des acides aminés naturels, la configuration de l'atome de carbone portant les substituants R¹ et R² peut être une configuration R, S ou un mélange racémique ;
*Cycl* est un groupe représenté par l'une ou l'autre des formules suivantes : 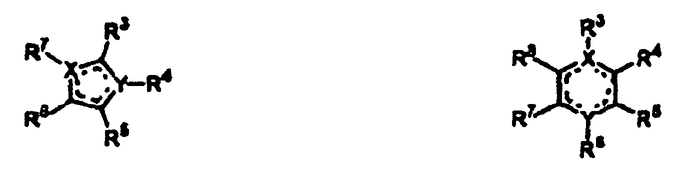 dans lesquelles :
R³ à R⁸ peuvent être identiques ou différents et représentent chacun un groupe alkyle facultativement substitué, cycloalkyle, alcényle, cycloalcényle, alcoxy, alkyloxy, alkylthio, cycloalcoxy, acyloxy, aryle, aryle substitué, hétéroaryle, hétéroaryle substitué, amino, amino substitué, phényle, et phényle substitué, un atome d'hydrogène, un atome d'halogène, un groupe cyano, un groupe nitro, un groupe thiol ou un groupe hydroxyle, l'un de R³ à R⁸ représente la liaison fonctionnelle avec la double liaison du coeur dicétopipérazine décrit ci-dessus, la liaison en pointillés représente une liaison choisie dans le groupe constitué par une liaison simple ou une liaison double et
X et Y peuvent être identiques ou différents et représentent chacun un atome de carbone ou un atome d'azote ou un atome d'oxygène ou un atome de soufre, les atomes de carbone, d'azote ou de soufre peuvent être facultativement oxydés ou substitués.

2. Procédé selon la revendication 1 sur un phosphinylglycinate supporté solide pour la préparation de membres uniques ou de groupes de déhydro-dicétopipérazines en phase solide, le phosphinylglycinate supporté solide étant représenté par la formule générale : 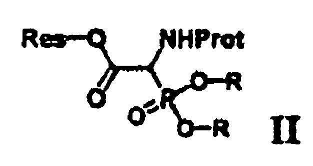 dans laquelle :
Res représente tout support polymère (résine),
Prot représente tout groupe NH-protecteur,
R représente des groupes alkyle ou alkyloxyalkyle.

3. Préparation d'un phosphinylglycinate supporté solide de formule (II) selon la revendication 2, par couplage du groupe carboxylate d'un phosphinylglycinate *N-*protégé et d'une résine à terminaison -OH en utilisant des conditions de couplage conventionnelles.

4. Utilisation du phosphinylglycinate supporté solide de formule (II) décrit dans les revendications 2 et 3, pour la préparation de déhydro-dicétopipérazines décrites dans la revendication 1.
